# EUROPEAN PATENT APPLICATION

(11) **EP 2 740 443 A1**
(43) Date of publication of application: **11.06.2014**
(21) Application number: 12819721.7
(22) Date of filing: 03.08.2012
(51) Int. Cl.: A61F 2/10, A61L 27/60

(54) **PREPARATION OF FIBRIN GEL FOR USE AS IMPLANT SYSTEM**

(30) Priority: 03.08.2011 CL 18702011
(71) Applicant: Instituto Para el Desarrollo Biotecnológico y la Innovación S.A., Viña del Mar (CL)
(72) Inventor: WEINSTEIN OPPENHEIMER, Caroline Ruth, Valparaiso (CL); BROWN GONZÁLEZ, Donald Irving, Valparaiso (CL); FUENTES CHANDÍA, Miguel Ángel, Valparaiso (CL); CERIANI FERNÁNDEZ, Ricardo Andrés, Valparaiso (CL); ALBORNOZ MÁRQUEZ, Fernando Antonio, Valparaíso (CL); ACEVEDO GUTIÉRREZ, Cristian Andrés, Valparaíso (CL)
(74) Representative: Stiebe, Lars Magnus
(86) International application number: PCT/CL2012/000039
(87) International publication number: WO 2013/016836

(57) **Abstract**

The present invention aims to generate a fibrin gel for cell proliferation and transport using the patient's own blood or compatible blood. The gel-preparation process consists in taking a quantity of blood from the patient, using sodium citrate as anticoagulant, separating off the citrated plasma by centrifugation, and resuspending the cells to be transported in the plasma. CaCl₂ is added to the resulting suspension to form the gel (either as an isolated coagulate or within a porous chitosan, gelatin or hyaluronic acid matrix), and the gel is cultured in an incubator to promote cell growth. The invention will be of use in cell growth for the purposes of implantation in cutaneous, cartilaginous, gingival and bone lesions, *inter alia,* and in the field of tissue engineering.

## Description

### BACKGROUND

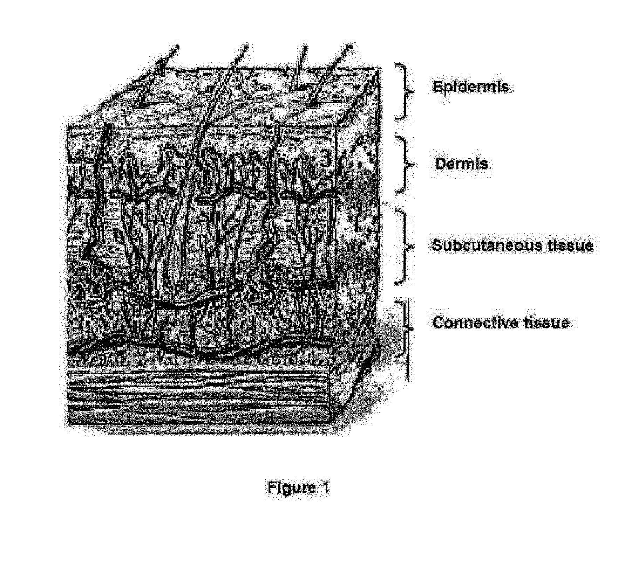

The skin is an organ consisting of three cell layers: epidermis, dermis and hypodermis, and by annexes: hair, nails, sebaceous and sweat glands (Castillo, 2001), as shown in Figure 1. The epidermis is the most superficial layer of the skin and the first protection barrier of the body from foreign substances. It is constituted by two groups of cells: keratinocytes or not dendritic cells, and dendritic cells (Merkel, Langerhans cells) and indeterminate cells (Navarrete, 2003). The epidermis in turn, is subdivided into 5 layers:
- Stratum germinativum or basal: as its name implies, this find germ cells needed for the regeneration of the epidermis layers. It is separated from the dermis by a thin sheet called basement membrane.
- Stratum spinosum: cells begin to accumulate a large number of desmosomes (or cellular bridges) on its outer surface, which gives a spinous or prickly characteristic.
- Granular layer: this layer cells accumulate keratohyalin granules. These granules contain lipids, which together with the desmosomes, help to form an impermeable barrier, which prevent the body fluid loss.
- Stratum Lucidum: represents the transition between the stratum granulosum and stratum corneum, and it can be clearly seen in the thick epidermis (palms of the hands and soles of the feet).
- Stratum Corneum: since the cells continue to accumulate (keratinization) keratohyalin granules, causes rupture of the Lysosomal membrane, and thus, the liberation of Lysosomal enzymes, causing cell death. The dead cells and senescent cells containing mature keratin, called corneocyte, form the Stratum Corneum. Gradually dead cells begin to separate from the rest, and subsequently lost, process known as scaling (Haake et al., 2001). Between the dermis and the epidermis, there is an area called dermal-epidermal junction, which has 4 main areas: plasma membrane from basal cell, lucid blade, blade dense and fibrous area (Hib, 2001).

### Dermis

The dermis is located under the epidermis, and is composed of connective tissue ground substance, forming together with glycosaminoglycans and fibrillar protein extracellular matrix, cell, and primarily fibroblasts, which are responsible for producing collagen and elastin, which give support and elasticity to the skin (Green, 1991). It also has cells of the immune system that help in defense against pathogens. The dermis is divided into two areas:

Papillary dermis: contains vascular networks that serve two important functions: the first is nourishing the avascular epidermis, and the second is feathers. The vascular network is interfingered in areas called dermal papillae. In the papillary dermis, we also found sensory nerve endings, as for example, the Meissner corpuscle.

Reticular Dermis: consists of a dense, irregular connective tissue. It is important because it gives skin firmness and elasticity. In addition, it serves as support annexes skin, glands and hair follicles (Haake et al., 2001).

### Hypodermis

Also called adipose panniculus or subcutaneous tissue. It is made by fat cells, which are known with the name of adipocytes, which are arranged in lobes separated by connective tissue called Septum and interlobular Septum (Navarrete, 2003). In the hypodermis are: the vascular network deep, lower portions of some hair follicles, acinar glands, eccrine and apocrine, Vatter-Puccini sensory corpuscles, sensitive to pressure changes and heat-sensitive Ruffini corpuscles.

### Skin lesions

Among lesions that may affect this organ, burns are one of the diseases that most often involve the urgency consultations, being the variety of trauma, which leaves in the patient, in the more usual way, serious and permanent sequelae (Aguayo, 1999). Globally, burns are a serious public health problem. There are more than 300,000 deaths each year only by fire. Death by fire product Burns, is among 15 causes of death in children and young adults ages 5 to 29 years. In addition, millions of people are disabled or suffer disfigurement of life (WHO, 2008). In a schematic way, one can say that in Chile, 50,000 people a year suffer some burn, 5,000 of them are hospitalized, and 500 die. Of this total, two-thirds are children, the highest risk group, along with the older than sixty years of age (Castillo, 2003). The main feature of burns injuries, is the loss of cutaneous cover, either partial or total. In the case of severe burns, there is loss of electrolytes and fluids, and infection of the skin that can trigger a systemic infection (CIGNA Health Corporation, 2008).

Burns, according to the damage caused to the tissue were traditionally classified in:
- grade 1 or erythematous, where the injury is very superficial and is regenerated in the span of a week without scarring (i.e. level of epidermis) grade 2, when they present bullae (blisters), where the lesion involves epidermis and superficial (papillary) Dermis layer, and is regenerated in a span of 8 to 14 days without scarring and third degree burns, where the lesion occupies the full thickness of the skin, namely epidermis, dermis and hypodermis, causing charring or a translucent white color in the skin, and cannot be regenerated by there is no skin elements to do so (Cienfuegos et al., 2003; Basin and Alvarez, 2003).

However, a more recent scheme has been described. The injuries that affect only epidermis are called surface. Burns, also involving the dermis, are classified as partial thickness burns, and they may be superficial or deep (the first affect the papillary dermis and the latter both papillary dermis as a lattice). Burns that extend through all layers of the dermis and subcutaneous tissue, are called Burns full thickness (Ehrenreich and Ruszczak, 2006). Skin ulcers are another type of frequent injury and difficult to treat. Occur, usually because of a venous stasis, diabetes, vasculitis, or arterial occlusion. They are classified by stages 1-6; stage 1 that where the ulcer is not really formed intact skin is simply flushed, and stage 6 that where there is destruction of the outer layers of the skin (Bolivar-Flores and Kuri-Harcuch, 2000). Venous ulcers, which represent between 80-90% of the total vascular ulcers can be found within skin ulcers. They are more common in women, with a ratio of 1-3. Chronicity and recurrence are its most relevant clinical features, half remain open over the nine months, 20 one-third of the initially healed (CIGNA Health Corporation, 2008) are up to two years, and a 10 to five, reappearing it, within the twelve months following his cure.

Venous disease is a precursor of venous hypertension (hypertension, venous ulcers, constitute the highest percentage out of the total of the vascular, 75 to 90%), and a risk factor for the subsequent development of ulcers on the lower extremities.

Venous ulcers of the lower extremities are transformed into a clinical problem, affecting between 1 and 2 of the Western population (Castillo et al., 2004). The decrease in circulation and Neuropathies, are the leading cause of diabetic foot ulcers, and can lead to amputation of the extremity (CIGNA Health Corporation, 2008). In its pathogenesis, initial alterations occur in the venous macrocirculation, mainly for reflux and occasionally by obstruction. These alterations cause venous hypertension, which affects the microcirculation, secondarily causing pericapillary fibrin deposits and activating cytokines and proteases that promote inflammation. This determines the appearance of lipodermatosclerosis, consisting of a fibrous tissue, with poor cellularity and irrigation and the consequent ulceration (Herouy et al., 2000).

### Wound healing

Wound healing, is a dynamic biological process that involves complex interactions of events cellular, molecular and biochemical (Lance, 2000). In this process, place interactions between the cells of the epidermis and the dermis, the extracellular matrix, controlled angiogenesis, and proteins derived from plasma, all coordinated by a number of cytokines and growth factors (Harding et al., 2002). This process has traditionally been divided into three distinct phases: inflammation, proliferation and remodeling (Schilling, 1976).

### Hemostasis and Inflammation

This stage occurs, immediately after the lesion or injury between days 4 and 6 produced. The first step is to stop the bleeding. There is contraction of the blood vessels damaged, and the endothelium and the area near platelets activate the intrinsic pathway of the coagulation cascade. The formed clot, is composed of collagen and platelet, Thrombin, fibronectin (Broughton et al., 2006). Trapped in the clot, mostly platelets, cells trigger the inflammatory response through the release of vasodilators and chemotactic and activation of the complement cascade (Clark, 1996). The fibrin clot, also serves as a prelude to invading cells like: neutral edges, monocytes, fibroblasts and endothelial cells (Kurkinen et al., 1980).

In the first stage of inflammation, predominantly neutrophils. They are attracted to the wound area by agents such as chemotactic: Interleukin 1 (IL-1), the tumor necrosis factor (TNF-α), factor (TGF-β) transforming growth, PF4 and bacterial products (Broughton et al., 2006;) Tamariz et al., 2002). Neutrophils removed bacteria and cellular debris from the wound, through the release of proteolytic enzymes that digest the bacteria and dead tissue (Broughton et al., 2006). The damaged extracellular matrix is also cleaned by Metalloproteinases matrix (MPP by its acronym in English), which are expressed by keratinocytes, fibroblasts, monocytes and macrophages in response to TNF-α (Abraham et al., 2000). Subsequently, the number of neutrophils decreases and these are replaced by macrophages. These activated macrophages, will act as mediators in angiogenesis (synthesizing endothelial growth factor vascular VEGF, FGF and TNF-α fibroblast growth factor), in the fibroplasia by synthesis of TGF-β, growth factor epidermal EGF growth factor derived from platelets PDGF, IL-1 and TNF-α and finally, synthesize nitric oxide (NO), through the activation of nitric oxide synthase inducible by IL-1 and TNF - α (Witte and snapper2002). Macrophages play a fundamental role, and mark the transition between the inflammatory phase, and the proliferative (Harding et al., 2002).

### Proliferative phase

At this stage occur epithelization, angiogenesis and the formation of a temporary matrix, and this stage runs from the 4th to 14th. The predominant cells at this stage, are fibroblasts and endothelial cells (Broughton et al., 2006). Epithelization is stimulated, in principle, by inflammatory cytokines (IL-1 and TNF-α). Fibroblasts, on the other hand, synthesize and secrete (KGF-1 and KGF-2) keratinocyte growth factor and IL-6, which encourages neighbouring keratinocytes migrate to the damaged area, to proliferate and differentiate into the epidermis (Smola et al., 1993;) Broughton et al., 2006).

Fibroblasts migrate to the wound site from nearby tissues, and become active (PDGF and EGF are main signals to the fibroblasts), so they begin to synthesize collagen, and proliferate to finally transform into myofibroblasts, which cause the contraction of the wound (Sage, 2001). For the formation of the temporary array, the fibroblasts (stimulated by PDGF) begin to synthesize, in addition to collagen, fibronectin, and proteoglycans as for example, hyaluronic acid (Harding et al., 2002;) Lynch et al., 1989). TGF-β, leads also to the fibroblasts to synthesize collagen type I, also decreasing the production of Metalloproteinases (MMP) matrix and increasing the production of proteins in cell adhesion (Goldman, 2004).

The keratinocytes, on the other hand, secrete VEGF (also secreted by macrophages, fibroblasts and platelets), which attracts endothelial cells located in the surroundings, thus beginning the formation of new capillaries. Endothelial cells also produce nitric oxide, which protects new tissue from the toxic effects of ischemia and damage by reperfusion, causing vasodilation of the endothelium (Broughton et al., 2006).

### Maturation and remodeling phase.

Clinically, the maturation and remodeling phase is perhaps the most important, and goes from the 8th until the year. The remodeling, or final stage is characterized by formation of the extracellular matrix, which is initially composed of fibrin and fibronectin. Then, fibroblasts begin to synthesize glycosaminoglycans, proteoglycans, and other proteins (Broughton et al., 2006).

This temporary array is then replaced by a more rigid and organized matrix composed mainly of collagen (in granulation tissue collagen type III reaches 30, while in the mature scar under 10). The proteinase present in matrix remodelling, are regulated by changes in the concentrations of TGF-β, PDGF, IL-1 and EGF.

MMP activity is also suppressed by MMP inhibitors produced by fibroblasts in tissue (Henry and Garner, 2003). The synthesis of collagen will continue for at least four to five weeks after that occurred the lesion (Diegelmann, 2003). The collagen in the scar, will never be as organized and structured (even after a year), as the collagen found in healthy skin. The strength and firmness of the skin will not be 100 % normal (Broughton et al., 2006). After the closure of the wound, remodelling of the resulting scar can be months or years, and leave as a result, a reduction in the number of cells and a decrease in blood flow in the scar tissue (Harding et al., 2002). In conclusion, a full-thickness injury healing, requires resurfacing from the limits of the wound, through the proliferation and migration of keratinocytes, through a temporary extracellular matrix, which is then converted into tissue granulation, which will be finally remodeled neodermis (Clark, 1996;) Tamariz et al., 2002).

### Dermal substitutes

The need for rapid closure of wound in extensive burns, made to develop methods of expansion and reproduction cells autologous or heterologous of the dermis and epidermis in vitro (Green et al., 1979) so, as today, it is possible to make a culture on number of different cell formulas (pure keratinocyte or mixed with fibroblasts). From a small biopsy, human skin can be grown in large amounts of three to four weeks and used as autografts, and may cover the total body surface area of an adult (Cuenca and Alvarez, 2003). The use of autografts and allografts of cultured epidermis, has been established as part of the therapy of the burned patient. In fact, the guides clinics of the great burning, produced by Chilean experts to guide the treatment within the framework of the explicit health guarantees, including the use of cultured keratinocytes (MINSAL, 2007). Clinical studies demonstrate that the allograft of human epidermis, grown in vitro, act as a dressing biologically active, to reduce the time of epithelization in second-degree burns deep, donor areas of autografts, dermabrasion, and ulcers by venostasis or diabetes (Sosa et al., 1999). So, as today, a series of dermal substitutes, are available as shown in table 1:

**Table 1: Different types of dermal substitutes (Dini et al., 2006).**

| **Dermal Substitutes** | **Composition** |
|---|---|
| Auto grafts | Skin taken from the same patient, or from patient cultured cells in a laboratory to obtain a thin layer (auto implants or grafts from epidermal cultures or auto implants from dermal cultures). |
| Alo grafts | Skin or cells taken from another person (fibroblasts and keratinocytes culture). |
| Xeno grafts | Skin or cells taken from another species (pigs). |
| Artificial skin | Biomaterials with or without molecules, living cells, genes or products from genes. |

Currently, a series of synthetic biomaterials that operate as extracellular microenvironments, imitating the regulatory features having natural extracellular matrix (ECMs) have been developed.

Biomaterials play a central role in modern strategies of regenerative medicine and tissue engineering, as a design of a biophysical and biochemical environment which leads cell behavior and functions. This can facilitate the restoration of structures or functions of the dysfunctional or damaged tissue.

Biomaterials, provide interim support interacting biomolecular with cells, guiding spatially and temporally complex multi-stage processes of formation and regeneration of tissues (Lütolf and Hubbell, 2005;) Bacakova et al., 2004). Therefore, the purpose of these biomaterials is mimic, to some extent, the processes that occur *in vivo.* Dermal substitute or ideal synthetic biomaterial, must have or comply with the following characteristics: absence of antigenicity, compatibility with tissue, absence of local or systemic toxicity, impermeability against exogenous organisms, rapid and sustained adherence to the surface of the wound, elasticity, to allow the movement of the tissue, resistance, should be translucent to allow direct observation of healing have a low cost, causing minimal discomfort to the patient, which reduces the time of healing, among others. (Ehrenreich and Ruszczak, 2006; Smith et al., 1988).

The therapeutic advances that have been made to the products developed by engineering in tissues, are based on three strategies:
the use of cells without matrix (such as autologous cell transplantation or stem cell therapy);
the use of synthetic polymers with or without growth factors and cytokines; and
the use of a three-dimensional matrix with cells in it (Jimenez and Jiménez, 2004).

There are a number of these experimental systems available. These range from matrix derived from cells or tissues (e.g.: Matrigel), hydrogels of synthetic polymer, to matrix composed of recombinant proteins. Bioinert surfaces are also been developed, using molecules such as protein albumin anti adhesive, hydrogels based on hyaluronic acid or poly (hydroxyl ethyl methacrylate), Poly, polyacrylamide, dextran and alcohol particularly polyethylene glycol (PEG) (Bacakova et al., 2004).

Thus, biomaterials derived from natural ECM, can be used as supports for transplanted cells, which will be subsequently implemented on injured tissue, and also to induce regeneration and remodeling *in vivo.* For example, collagen and fibrin as arrays, are clinically established and approved by the FDA (Food and Drug Administration), in the treatment of burns, for the healing of wounds and tissue repair, respectively (Lütolf and Hubbell, 2005;) Van Dorp et al., 1998). Fibrin products, contain two components isolated from human plasma: Fibrinogen and Thrombin. The combination of these two components gives as a result, the formation of a fibrin clot, which is used to achieve hemostasis, and the closure of the wound in surgical processes (Geer et al., 2002).

In a study conducted by Cox et al., 2004, analyzed the fibrin clot as a means of transport of human dermal fibroblasts, and also assessed, proliferation and migration of these in different formulations of clots, varying the concentrations of Fibrinogen and Thrombin, coming to the conclusion that the variation in the concentrations of both components, affected the behavior of the fibroblasts in three-dimensional fibrin clots.

The first commercially available dermal substitute, used similar collagen based on bovine collagen and Chondroitin-6-sulfate with an outer covering of Silastic® (Burke J et al., 1981).

Dermagraft® is a modification of the previously composed dermal substitute, where instead of bovine collagen, fibroblasts obtained from the foreskin of newborn infants, are cultivated on a nylon mesh and covered with an outer silicone layer (Dermagraft Transitional Covering). It is designed to cover the dermal layer of the skin and to stimulate an improvement in the wound healing process (Mansbridge et al., 1998).

There are also other types of dermal substitutes, which incorporate both epidermal components as dermal (mixed or composite substitutes). The first composite substitute was developed by Ortec International Inc., and was a member of fibroblasts and keratinocytes of newborns, grown on a porous matrix criss cross with bovine collagen type 1. However, the use of this substitute is limited, since the FDA has approved it only for two indications: the treatment of reconstruction of hand in patients who suffer from Epidermolysis Bullosa recessive, and as aid in the healing of donor areas of autografts in burned patients (Pham et al., 2002;) Eisenbud et al., 2004). Grañskin (Apligraj®) it is also a joint replacement, but with multiple applications. It was developed for the coverage of full thickness wounds. It is a graft mixed allogeneic consisting of human epidermal cells, human fibroblasts and bovine collagen type 1, which delivers 4 components: epidermal keratinocytes, a stratum corneum well differentiated, matrix extracellular and viable allogeneic dermal fibroblasts (Pham et al., 2002; Vêves et al., 2001). On the other hand, Mylan Laboratories, Inc., developed Biobrane, which is a biosynthetic product, consisting of an ultra-thin semipermeable silicone film, with nylon fibers partially embedded in the film, linked chemically to collagen from porcine (UDL Labs, 2008). It is used, mainly in patients with partial thickness burns and wounds relatively fresh (24-48 hours) (Gerding et al., 1990). Another product of Bioengineering, which was approved by the FDA in 1997, but has a high cost, is TransCyte ®, composed of newly born human fibroblasts, grown under aseptic conditions on the component of Biobrane nylon mesh. Fibroblasts secrete into the mesh, components of the extracellular matrix like fibronectin, collagen type 1, decorin and growth factors linked to the matrix (Ehrenreich and Ruszczak, 2006;) Noordenbos et al., 1999). However, in most of the cases, it is difficult to argue the use of these expensive products (for example in donor areas of skin) when there is more economic and effective alternatives that are already available, like Biobrane and conventional implants for wound coverage. In extensively burned patients, where the donor areas of autografts are limited or are not appropriate (to use them requires two or more weeks), could justify the use of biomaterials that will provide a temporary wound coverage, thus, promoting a faster and better quality healing (Sosa et al., 1999; Bar - Meir et al., 2006).

There are, on the contrary, lower cost biomaterials, and which have been used widely in tissue engineering, as in the case of the Chitosan and hyaluronic acid. The first is a complex, non-toxic, and biodegradable carbohydrate derived from chitin, which has shown mucoadhesive activity, this makes it an excellent Hemostatic agent (Wedmore et al., 2006). Also has other biological properties (antibacterial and antifungal) and affects the macrophages function, which helps fast healing. Also, has ability to stimulate cell proliferation and tissue histoarchitectonics organization (Paul and Sharma, 2004;) Fukasawa et al., 1992). On the other hand, the hyaluronic acid (HA), is a glycosaminoglycan which is normal skin in the intercellular spaces of the skin, except in the granular layer and stratum corneum. Besides being a matrix in which the cells are embedded, it was discovered that it has numerous functions on the skin: can retain water in the tissues, due to this change the volume and compressibility dermal; It can also improve proliferation and cell differentiation and the repair of damaged tissue (Juhlin, 1997;) Laurent and Fraser, 1992).

The developed system is called integrated, because the cells are not located on the surface of the support but embedded in it (Young et al., 2006). This system has proven an excellent adhesion to the area of the lesion and, on the other hand, no observed toxicity reactions to the system components.

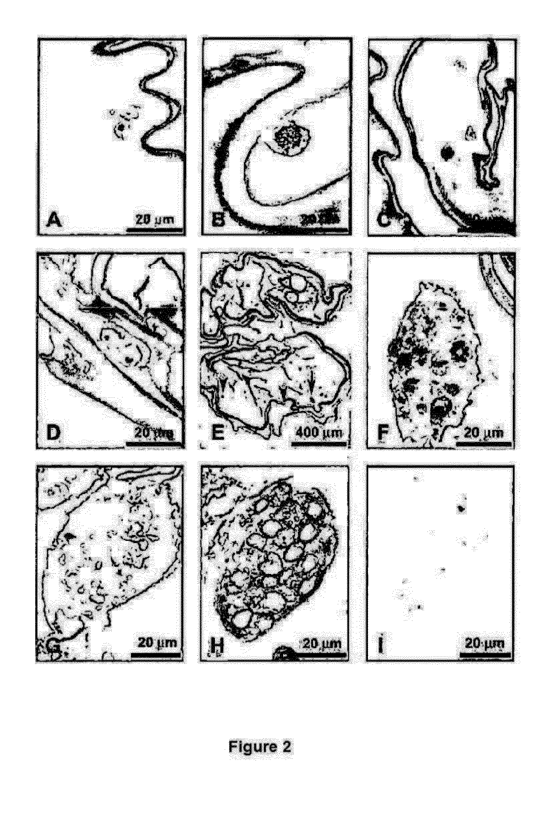

In addition, studies published by the own inventors of this application are encapsulated into fibrin, skin cells to exhibit a growth pattern different from cells cultured in conventional culture bottles (Figure 2). A problem found by the research group, has been the high cost of clinical grade materials, that limit the risks of contracting infectious diseases with these materials and that are essential to implement the its in humans. Among these materials, the Fibrinogen and Thrombin has been that most exceed in price by using equivalent approved for human use. For this reason, there is a need to generate Thrombin autologous, using plasma of the patient as a way of reducing costs. This creates the requirement of studying the conditions to create an optimal clot that allow cell growth, especially to the interior of the SIL.

### Detailed Description of the Invention

The technical issue raised in this application consists of providing a product and system preferably autologous allowing immobilize, proliferate and conveyed cells within a matrix for the purpose of tissue regeneration.

The search of biomaterials that cell growth is a fertile ground. Required materials that mimic the extracellular cell with its three-dimensional features. Within these biomaterials collagen, gelatin, alginate and other materials have been used. Among these, the use of fibrin has shown extraordinary properties to promote the growth of skin cells. However, in the clinical application of these cultures for the treatment of skin lesions is with the disadvantage that only clinical grade materials should be used. The latter exist and are called Tissucol® and Beriplast® in the clinic is used as fibrin sealants in different surgical procedures. Found these products maintain the properties of cell growth promoting detected with the combination of Thrombin and Fibrinogen level *in vitro* research. However the above the cost of these products, given the complex microbiological analysis that require, makes them incompatible with the possibility of creating a commercial product containing fibrin, and cover large areas of damaged skin. Materials that are safe for human beings, free of animal components and risk of transmission of known and emerging diseases are also required. A product of autologous origin will always be more secure from the microbiological standpoint than one heterologous, since although this polluted, will return to the same host. On the other hand, from the immunological point of view, antibodies and other proteins present in it will be those already existing in the individual, eliminating the possibility of immune rejection. The present invention is directed to generate a fibrin gel for proliferation and cell conduction from the patient's own blood or blood compatible. The blood rate of 200 µl per cm of implant to prepare is taken using sodium citrate as an anticoagulant in a reason of 0.09 g per mL of blood. Plasma citrated is separated by centrifugation. The cells are then suspended again in this plasma. To form the gel either as isolated clot inside a porous matrix is to add a CaCl2 solution. The product thus obtained is of much lower cost than commercial "fibrin glue" or "fibrin sealant" and for being an autologous product, the risk of infection and immune rejection is avoided, and although it may be difficult to obtain large volumes of blood from a patient, this can be overcome using compatible blood. This system has been used to treat skin lesions with positive results. The application will be for purposes of cell growth in skin lesions implants, cartilage, osseous and gingival lesions among others. It can be anticipated that any application in the field of tissue engineering can use this technology.

The novelty lies in the incorporation of the cells into a fibrin clot using plasma instead of purified fibrin and thrombin. The fibrin glues contain both components and some others from blood, but they are no equivalent to plasma since they are purified. Also they incorporate additives, such as protease inhibitors that are not present in plasma.

All shown references are ways for generating the fibrin clot, but not ways for integrating cells in a fibrin clot with purposes of tissue engineering.

There are many publications that support the potential of growing cells in fibrin clots. For this, commercial fibrinogen and thrombin are acquired or commercial preparations known as fibrin glues are used, the most popular being Tissucol (Baxter) or Beriplast (CSL Behring). These products allow the cells to be suspended in thrombin and then fibrinogen is added for making a cell containing clot. The problem is that for doing this with clinical and commercial purposes a lot of money should be expended because the products of clinical grade are very expensive. It is also difficult to be 100% sure that the clot will not transmit any virus or unknown microorganism for which there are no screening tests with appropriate sensitivity.

Thus, the idea of generating fibrin for cell growth from the patient's own blood to form the gel, either as an isolated clot or within a porous matrix, arises.

According to the above, the product generated in the present invention has as main advantages that it has much lower cost than the commercial "fibrin glues" and for being and autologous product, it eliminates the risk of infection and immune rejection. On the other hand, the disadvantage that it can be difficult to obtain large volumes of blood from a compromised patient, is solved using compatible blood.

For the support of the invention, a number of clinical trials have been developed in which the cells are incorporate into a porous matrix by the use of autologous blood. This system has been used to treat skin lesions with positive results.

An object of the present invention is the application for the purpose of cell growth in implants for skin, cartilage, gingival and bone lesions, among others.

The patent literature shows homologous products that differ of the invention in fundamental aspects. A description of the most relevant known in patents follows:
Beretta & Grippi in the patent applications numbers US 2009258056 and WO 2007021344 and Beretta & Lodi in patent number US 6,368,298, developed methods for regenerating tissue in a living organism, which include contacting the affected area with a net of solid fibrin containing platelets that release growth factors. The gel is a-cellular and contains platelets derived from autologous blood.

Similarly, Baugh & Lim in patent applications numbers US 2005152886 and US 2002159985, and in the patent number US 6,444,228, and Hirsch & Johnston in patent number EP0820314, describe methods for obtaining autologous fibrin sealant, which does not contain incorporated cells but only platelets. The patent number US 5,185,001 by Galanakis D., discloses a kit which contains all the elements to produce a fibrin clot from autologous plasma, in order to use it to induce hemostasis (clotting in a wound), but no reports the inclusion of cells in it.

Moreover, in the Chilean patent application CL 1439-2002, obtaining of a dermal substitute from plasma, using calcium chloride during gelation is described, but just as same as previously disclosed inventions, the cell component from a culture is not incorporated. It is mentioned that it allows the invasion by cells and blood vessels, but it refers to those of the wound bed and not exogenously provided.

Additionally, there is a significant amount of scientific articles, as well as some patents already mentioned, pursuing the research of the same technical problem. However, they are all ways to generate the fibrin clot and not ways for integrating the cells into a fibrin clot with tissue engineering purposes.

### Examples

The examples below indicated, are incorporated by way of illustration only, to facilitate understanding of the invention, and not meant to limit in any way the scope of the requested claims.

### Example 1: Obtaining and culture of human fibroblasts

Human fibroblasts are obtained from foreskins of surgeries due to phimosis in children under 6 years. The biopsy is washed in a sterile Petri dish with 5 ml of phosphate buffered saline solution, 0.1 M pH 7.4 (PBS) (GIBCO®),containing a mixture of penicillin/streptomycin antibiotics 100 U/ml /100 µg/ml, Invitrogen®) and amphotericin B 250 µg/ml (Fungizone®, Invitrogen). To obtain separate fibroblasts and keratinocytes, the sample is incubated in trypsin 0.5%, 5.3 mM EDTA (GIBCO®) or 30 minutes at 37°C in incubator Thermo Forma®. Subsequently, using sterile forceps, the dermis and the epidermis are mechanically separated. The dermis is treated for 20 min with collagenase type I (Invitrogen®) 2 mg/ml at 37° C. Then, the obtained cell suspension is centrifuged and the precipitated obtained is washed with PBS to remove the enzyme. Its cell viability is determined by stain exclusion with trypan blue. This is accomplished by microscopic examination of an aliquot of cell suspension mixed with trypan blue stain in a 1:1 ratio. Then this suspension is placed in a Neubauer chamber and viable cells are counted by observation in inverted microscope Lieder ®. Human fibroblasts obtained after enzymatic digestion, are re-suspended in Eagle culture medium and modified by Dulbecco (DMEM, GIBCO®) supplemented with 10% of fetal bovine serum ((INVITROGEN®), 50% Ham F12 medium (GIBCO ®) and 10 ml/ml Biomyc 1 (Biological Industries ®) in culture flasks T-25 (Falcon ®), that are incubated in an incubator thermoformed, in a humidified atmosphere with 5% CO₂ and a temperature of 37°C. 50% medium F12 of Ham (GIBCO®) and 10 µl\ml of Biomyc 1 (Biological Industries@) in culture flasks T-25 (Falcon@), that are incubated in an incubator ThermoForma, in a humidified atmosphere with 5% of CO₂ and a temperature of 37°C.

### Example 2: Obtaining and evaluation of human origin fibrin clots

Obtaining the plasma. The plasma is separated from whole blood collected into tubes containing sodium citrate at 3.2%, centrifuged at 3000 rpm for 5 minutes. The separated plasma in sterile conditions under bio-safety hood, stored at -20°C until use.

Clotting test by visual observation: Initial tests of clotting, are performed in microcentrifuge tubes by testing various concentrations of calcium, named formulations (F). For this, a constant volume of citrated plasma 100µl is used with different volumes of dilutions of CaCl₂, to obtain different final concentrations of the same, generating 30 formulations as illustrated in Table 2:

**Table 2. Clots formulations according to the CaCl₂ concentrations.**

| CaCl₂ Concentration (mM) | CaCl₂ Volume (µL) | | | | | |
|---|---|---|---|---|---|---|
| | **25** | **50** | **75** | **100** | **150** | **200** |
| **10** | F1 | F2 | F3 | F4 | F5 | F6 |
| **20** | F7 | F8 | F9 | F10 | F11 | F12 |
| **30** | F13 | F14 | F15 | F16 | F17 | F18 |
| **40** | F19 | F20 | F21 | F22 | F23 | F24 |
| **50** | F25 | F26 | F27 | F28 | F29 | F30 |

After preparing the 30 formulations, these are incubated with the constant volume of plasma at 37°C for 20 minutes. They are visually evaluated, whether is coagulation in each formulation. Furthermore, each clot is removed from the microcentrifuge tube with forceps, for evaluation of its mechanical strength, and thus the best 6 formulations are selected, based on the existence of clot and the greatest mechanical strength. For the determination of the clotting time a clot timer (BBL Fibrosystem) is used, which allows to determine, more precisely, the time in which the clot forms. This test is performed only in the 6 preselected formulations in the preliminary stage. The stability of the clot is determined in 6 clots of the formulations selected with culture medium (DMEM, GIBCO ®), supplemented with 10% of fetal bovine serum(INVITROGEN®), 50% medium F12 of Ham (GIBCO®). Subsequently, it is incubated at 37 °C. The degree of disintegration is evaluated visually on day 3 (Figure 2).

### Example 3. Preparation of the polymer matrix

Preparation of base polymer: For the base polymer, the following solutions are prepared: gelatin 1% w/v, chitosan 2% w/v in acetic acid 1% v/v, and hyaluronic acid 0.01% w/v according to the following protocol:
The gelatin solution with chitosan and hyaluronic acid is mixed, and homogenized for 30 minutes with magnetic stirring at 50 °C.

Subsequently, the mixture is poured into Petri dishes that are refrigerated at 4°C until the formation of gel. Then, the gel is frozen slowly at -20°C for 8 hours. Then, the polymer is carried to -80°C for 8 hours.

The polymer is slowly immersed in liquid nitrogen for 3 minutes. Finally, it is lyophilized for 48 hours in Liobras LT01 lyophilizer.

Reticulation: For the base polymer reticulation, the following procedure is performed:
The lyophilisate is immersed in MES 50 mM (2-morpholinoethane sulphonic acid), for 30 minutes. Then, this solution is discarded and reticulant solution composed by MES 50 mM, EDC 30 mM (1-ethyl-[3,3-dimethylaminopropyl] carbodiimide) and NHS 8 mM (N-hydroxysuccinimide) are added. It is left to react for 2 hours.

Then it is washed with ethanol and frozen.

Once frozen, it is immersed in liquid nitrogen for 3 minutes.

### Finally, it is lyophilized for 24 hours.

### Example 4: Study of cell growth and proliferation for the selected surfaces

For this experiment, the technique is MTT [(3-4,5-dimethylthiazolyl-2) - 2,5-diphenyltetrazolium bromide] was used, which allows quantification of viable cells. This technique is based on the reduction of MTT, only by those metabolically active cells, in a compound called "formazan" a soluble purple blue crystal, which can be quantified by spectrophotometry at 540 nm.

MTT assay: Obtaining fibroblasts to this test is performed as follows:
Culture flasks T-75 (Falcon ®) with fibroblasts are washed with PBS, and then treated with trypsin. The cell suspension is placed in a 50 ml disposable centrifuge tube, and centrifuged at 3000 rpm for 5 minutes. Then, the precipitate obtained is re-suspended in order to verify the number of viable cells.

Once realized the cell counting, the suspension is centrifuged again, and a portion of the cells are re-suspended in culture medium (DMEM, GIBCO®), supplemented with 10% of fetal bovine serum (INVITROGEN®), 50% of medium F12 of Ham (GIBCO®) and a mixture of penicillin/streptomycin antibiotics (100 U/ml/ 100µg/ml), INVITROGEN®), and the other in plasma. This assay is performed in 96 flat bottom wells (Falcon ®), where the proliferation of fibroblasts is evaluated, using different surfaces for its cultivation. The assay is performed from day 0 to day 3, i.e. at 0, 24, 48 and 72 hours, and each condition is evaluated in triplicate with its level control for each day. Also, only the two best previously selected formulations are considered for testing.

Detailed below is the MTT assay to examine the proliferation of fibroblasts on different surfaces:

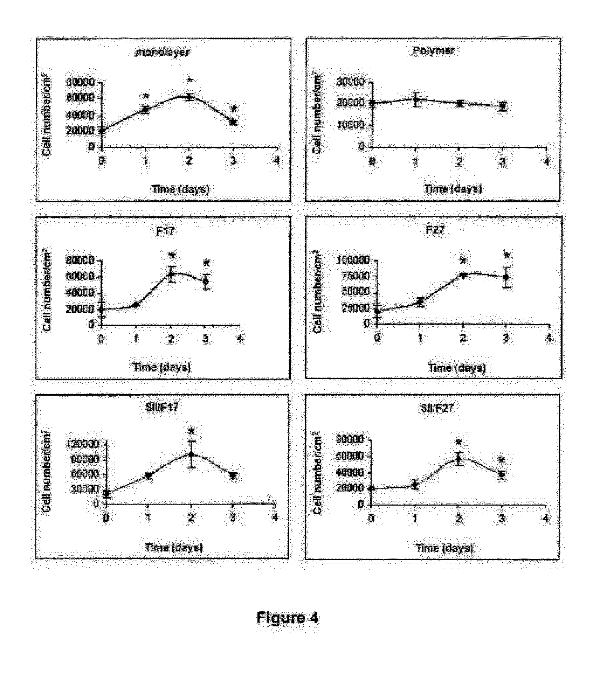

### Experimental conditions (Figure 4):

Monolayer: human fibroblasts re-suspended in medium (5x10⁶ cells per well), are cultured directly in the well with 150µl of culture medium.

Polymer Matrix: the polymer matrix is placed in each well and the re-suspended human fibroblast are delivered in medium (5x10⁶ cells per well). Culture medium is added until complete 150µl.

Clot F17: 30µl of plasma with humans fibroblasts are deposited (5x10⁶ cells per well), and to this 45µl of CaCl₂ 30 mM is added. Is incubated at 37°C until the clot formation and culture medium is then added until complete 150µl.

Clot F27: 30µl of plasma with humans fibroblasts are deposited (5x10⁶ cells per well), and to this 22.5µl of CaCl2 50 mM is added. Is incubated at 37 °C until the clot formation, and the culture medium is then added until complete 150µl.

SU with F17: on the polymer matrix, 30µl of plasma with human fibroblasts (5x10⁶ cells per well) and 45µl of CaCl₂ 30mM are deposited. Is incubated at 37°C until clot formation, and then the culture medium is added until complete 150µl.

SU with F27: on the polymeric matrix, 30µl of plasma with human fibroblasts (5x10⁶ cells per well), and 22.5 µl of 50 CaCl₂ mM are deposited. Is incubated at 37 °C until clot formation, and then the culture medium is added until complete 150µl.

MTT procedure: Once assembled the experiment with the different cell culture conditions, MTT assay for day 0 (0 hours) is performed to establish values of cell viability at the time of the beginning of the experiment. This procedure is subsequently repeated after 24, 48 and 72 hours. The procedure consists of:
Add 50 µl of MTT solution to each well, corresponding to the evaluation day and incubate at 37°C for 4 hours.

Subsequently, each solution in the wells is removed and transferred into the corresponding microcentrifuge tubes for each sample.

150µl of trypsin 10% is added to each well. The plate is again lead to the incubator at 37°C for 1 hour.

- Finally, 300 µl of lysis buffer are added into each well (for the monolayer 400µl), vigorously pipetted, and the solution is transferred to each microcentrifuge tube (this, in two steps, first add 100µl or 200µl, as appropriate, and in a second step add the remaining 200µl).

The tubes are maintained at -20°C until photometric quantification. To do this, microfuge tubes with samples of each day are thawed.

After thawing the tubes, these are agitated vigorously by vortexing.

Then, ultrasound is applied for 30 minutes.

The tubes are homogenized again by vortexing.

Ultrasound is reapplied for 30 minutes.

Subsequently, centrifuged at 14,000 rpm for 15 minutes.

Carefully, particle-free supernatants from each tube are extracted and 200µl of each sample are deposited into a 96 wells plate.

Finally, the absorbance is read on plate reader ELISA SENSISCAN (MERK®) at 540 nm.

### REFERENCES

### Patents

1. SYSTEM AND METHOD FOR PREPARING AUTOLOGOUS FIBRIN GLUE. Inventors: BERETTA ROBERTO; GRIPPI NICHOLAS A Applicant: BERETTA ROBERTO; GRIPPI NICHOLAS A. Publication info: JP2010115507 (A) - 2010-05-27, Priority Date: 2002-01-15.
2. SYSTEMS AND METHODS FOR PREPARING AUTOLOGOUS FIBRIN. Inventor: BERETTA ROBERTO [IT] ; GRIPPI NICHOLAS A [US] Applicant: CASCADE MEDICAL ENTPR LLC [US]. Publication info: US2009203613 (Al) - 2009-08-13. Priority Date: 1997-06-24.
3. SYSTEMS AND METHODS FOR PREPARING AUTOLOGOUS FIBRIN GLUE. Inventor: BERETTA ROBERTO [IT]; GRIPPI NICHOLAS A [US]. Applicant: CASCADE MEDICAL ENTPR LLC [US]. Publication info: US2009258056 (Al) - 2009-10-15. Priority Date: 1997-06-24.
4. SYSTEMS AND METHODS FOR PREPARING AUTOLOGOUS FIBRIN GLUE. Inventor: BERETTA ROBERTO [IT]; GRIPPI NICHOLAS A [US]. Applicant: CASCADE MEDICAL ENTPR LLC [US] ; BERETTA ROBERTO [IT] (+1). Publication info: WO2007021344 (Al) - 2007-02-22. Priority Date: 2005-08-17.
5. AUTOLOGOUS FIBRIN SEALANT AND METHOD FOR MAKING THE SAME. Inventor: BAUGH ROBERT F [US] ; LIM LISA M [US] (+2) Applicant: MEDTRONIC INC. Publication info: US2005152886 (Al) - 2005-07-14, US7811607 (B2) - 2010-10-12. Priority Date: 1996-04-30.
6. AUTOLOGOUS FIBRIN SEALANT AND METHOD FOR MAKING THE SAME. Inventor: BAUGH ROBERT F [US] ; LIM LISA M [US] (+2) Applicant: BAUGH ROBERT F, ; LIM LISA M, (+3). Publication info: US2002159985 (Al) - 2002-10-31, US6830762 (B2) - 2004-12-14 Priority Date: 1996-04-30.
7. PREPARING AUTOLOGOUS FIBRIN GLUE. Inventor: BERETTA ROBERTO [IT]; LODI SERGIO [IT]. Applicant: BERETTA ROBERTO [IT]. Publication info: US6368298 (B1) - 2002-04-09. Priority Date: 1997-06-24.
8. SINGLE USE SYSTEM FOR PREPARING AUTOLOGOUS PLASMA AND FIBRIN GEL. Inventor: WHITMORE ELAINE [US]. Applicant: WHITMORE ELAINE. Publication info: US6197194 (B1) - 2001-03-06. Priority Date: 1995-03-24.
9. AUTOLOGOUS FIBRIN SEALANT AND METHOD FOR MAKING THE SAME. Inventor: BAUGH ROBERT F [US] ; LIM LISA M [US] (+2) Applicant: MEDTRONIC INC [US]. Publication info: US6444228 (B1) - 2002-09-03. Priority Date: 1996-04-30.
10. PREPARATION OF AUTOLOGOUS PLASMA AND FIBRIN GEL. Inventor: WHITMORE ELAINE [US]. Applicant: JOHNSON & JOHNSON MEDICAL [US]. Publication info: US5935437 (A) - 1999-08-10. Priority Date: 1995-03-24.
11. PROCESS OF PREPARATION OF AN AUTOLOGOUS FIBRIN GLUE. Inventor: TARANTINO FLAVIO [IT] ; BENINCASA CARLO [IT] (+2) Applicant: TARANTINO FLAVIO [IT] ; BENINCASA CARLO [IT] (+2) Publication info: W0981 1925 (Al) - 1998-03-26. Priority Date: 1996-09-18.
12. AUTOLOGOUS FIBRIN GLUE AND METHODS FOR ITS PREPARATION AND USE. Inventor: HIRSH JACK [CA]; JOHNSTON MARILYN [CA] (+1). Applicant: HAMILTON CIVIC HOSPITALS RES [CA]. Publication info: EP0820314 (Al) - 1998-01- 28. Priority Date: 1995-04-06.
13. METHOD OF PREPARING AUTOLOGOUS PLASMA FIBRIN AND APPLICATION APPARATUS THEREFORE. Inventor: GALANAKIS DENNIS K [US]. Applicant: UNIV NEW YORK STATE RES FOUND [US]. Publication info: US5185001 (A) - 1993-02-09.
   Priority Date: 1990-01-18.
14. METODO of OBTENCION of SUSTITUIDO DERMICO A PARTIR of FIBRINA. S, CL 200201439. Inventor: ABELARDO SIMON OSVALDO MEDINA DIAZ. Solicitante : ABELARDO SIMON OSVALDO MEDINA DIAZ.

### Scientific Literature

1. Tomoko Ichiyanagi, Kouki Anabuki, Yoji Nishijima, Hirohisa Ono,* Isolation of mesenchymal stem cells from bone marrow wastes of spinal fusión procedure (TLIF) for low back pain patients and preparation of bone dusts for transplantable autologous bone grañ with a serum glue. BioScience Trends. 2010; 4(3): 110-118.
2. Aleksandra Wysocka, Karolina Mann, Henryk Bursig, Juliusz Dec, Tadeusz S. Chondrocyte suspensión in fibrin glue. Gaz'dzik Cell Tissue Bank 2010; 11: 209-215.
3. David M. Dohan Ehrenfest, Pierre Doglioli, Giuseppe M. of Peppo, Marco Del Corso, Jean-Baptiste Charrier. Choukroun's platelet-rich fibrin (PRF) stimulates in vitro proliferation and differentiation of human oral bone mesenchymal stem cell in a dose-dependent way. Arc. Oral Biol 2010; 55 :185 - 194.
4. García Guevara et al. Aplicación of fibrina rica en plaquetas (frp) posterior a extracciones dentarias. The preliminary program for iadr Venezuelan división annual meeting. November 15-16, 2010.
5. Kojun Okamoto et al. The use of autologous fibrin glue for the treatment of postoperative fecal fistula following an appendectomy: report of a case. Surg Today, año? 33(7):, 550 - 552.
6. Kenjiro Nakama et al. Use of autologous fibrin sealants to treat ganglion cysts: a report of two cases. J Orthop Surg 2010; 18(1): 104- 106.
7. Aparecida Machado of Moraes, Joyce Maria Annichino-Bizzacchi, ana Beatris Rodrigues Rossi. Use of autologous fibrin glue in dermatologic surgery: application of skin graft and second intention healing. Sao Paulo Med. J. 1998; 116 (4).
8. Vivostat® autologous fibrin sealant. http://www.vivostat.com/composite-41.htm. (Ingreso mayo 19 of 2011).
9. Abraham D, Shiwen X, Black C, Sa S, Xu Y y Leask A. Tumor necrosis factor alpha suppresses the induction of connective tissue growth factor by transforming growth factor-beta in normal and scleroderma fibroblasts. J. Biol. Chem. 2000. ; 275(20): 15220 - 15225.
10. Acevedo C, Weinstein C, Brown D, Huebner H, Buchholz R y Young ME. 201 1. A mathematical model for the design of fibrin microcapsules with skin cells. Bioprocess Biosyst Eng. 2009; 32(3):341-51.
11. Aguayo B. Manejo inicial of las quemaduras. Rev. Chil. Pediatr. 1999; 70(4): 337 - 347.
12. Bacákova L, Filová E, Ryácek F, Svorcík V, Stary V. Cell adhesión on artificial materials for Tissue Engineering. Physiol. Res. 2004; 53 (Suppl. 1): S35 - S45.
13. Bar-Meir E, Mendes D y Winkler E. Reviews: Skin substitutes. IMAJ. 2006; 8(3): 188 -191.
14. Bolívar-Flores Y y Kuri - Harcuch W. Cure of acute, chronic, and complicated leg ulcers with frozen allogenic human epidemial cultures. Home Health Care Consultant. 2000; 7(4): 1 1-16.
15. Bailey J, Ollis D. 1986. Biochemical Engineering Fundamentáis. 2a Edición. McGraw - Hill.
16. Broughton G, Janis J, Attinger C. The Basic Science of Wound Healing. Plast. Reconstr. Surg. 2006; 1 17 (Suppl): 12S - 34S.
17. Burke J, Yannas I, Quinby W, Bondoc C y Jung W. Succesful use of physiologically aceptable artificial skin in the treatment of extensive burn injury. Ann Surg. 1981 ; 194(4): 413-428.
18. Castillo P. La piel como fuente of malignidad. Rev. Chil. Pediatr. 2001 ; 72(5): 466 - 472.
19. Castillo P. Quemaduras. Conceptos para el médico general. Cuad. Cir. 2003; 17(1): 58 - 63. ISSN 0718 - 2864.
20. Castillo P, Sagúes R, Urrea C, Bardisa J, López A. Colgajo sural en úlceras venosas crónicas of piernas. Rev. Chil. Cir. 2004; 56 (5): 475 -480.
21. Cienfuegos R, Sierra E, Juárez E, Kuri-Harcuch W. Aloinjertos of epidermis cultivada para áreas donadoras of piel y lesiones of espesor total en pacientes politraumatizados. An Med Asoc Med Hosp ABC. 2003; 48(2): 84 - 88.
22. CIGNA Health Corporation. 2008. Cigna Healthcare Coverage Position. Tissue - Engineered Skin Substitute and Growth Factors. N° 0068: 1 - 40.
23. Clark R. 1996. Wound repair: overview and general considerations. En Clark, R (ed). The molecular and cellular biology of wound repair. Plenum, New York, pp 3 - 50.
24. Cox S, Colé M y Tawil B. Behavior of human termal fibroblasts in Three - Dimensional fíbrin clots: dependence on fibrinogen and thrombin concentration. Tissue Eng. 2004; 10 (5 - 6): 942 - 954.
25. Cuenca J, Alvarez C. Injertos en malla 1 :6 cubiertos con aloinjertos of epidermis cultivada en áreas cruentas por quemadura. Cir. Plast. 2003; 13(1): 13 -17.
26. Currie L, Sharpe J y Martin R. The use of fíbrin glue in skin grafts and Tissue - Engineered skin replacement: A review. Plast. Reconstr. Surg. 2001 ; 108: 1713 - 1726.
27. Diegelmann, R. Analysis of collagen synthesis. Methods Mol. Med. 2003; 78: 349.
28. Dini V, Romanelli M, Piaggesi A, Stefani A y Mosca F. Cutaneous Tissue Engineering and lower extremity rounds (Part 2). Int J Low Extrem Wounds. 2006; 5(1): 27 - 34.
29. Ehrenreich M. y Ruszczak Z. Tissue - engineering wound coverings. Important options for the clinician. Acta Dermatoven APA 2006; 15(1): 5 - 11.
30. Eisenbud D, Huang N, Luke S, Silberklang M. Skin substitutes and wound healing: current status and challenges. Wounds. 2004; 16(1): 2 - 17.
31. Fukasawa M, Abe H, Masaoka T. The hemostatic effect of deacetylated chitin membrane on peritoneal injury in rabbit model. Surg. Today. 1992; 22(4): 333 - 338.
32. Geer D, Swartz B. y Andreadis S. Fibrin promotes migration in a Three - Dimensional in vitro model of wound regeneration. Tissue Eng. 2002; 8(5): 787 - 797.
33. Gerding R, Emerman C, Effron D, Lukens I, Fratianne R. Outpatient management of partial - thickness bums: Biobrane versus 1% silver sulfadiazine. Ann Emerg Med. 1990; 19(2):121 - 124.
34. Goldman, R. Growth factors and chronic wound healing: Past, present, and future. Adv Skin Wound Care. 2004; 17(1): 24 - 35.
35. Green H, Kehinde O y Thomas J. Growth of cultured human epidermal cells into múltiple epithelia suitable for grafting. Proc. Nati. Acad. Sci. U S A. 1979; 76(11): 5665 - 5668.
36. Green H. Cultures cells for the treatment of disease. Sci. Am. 1991 ; 265(5):96 - 102.
37. Haake A, Scout G, Holbrook K. 2001. Structure and function of the skin: overview of the epidermis and dermis. En: The biology of the skin.
38. Freinkel R, Woodley D. The parthenon publishing group. pp: 19 - 45.
39. Harding K, Morris H, Patel G. Healing Chronic Wounds. BMJ 2002; 324: 160 - 163.
40. Henry G y Garner W. Inflammatory mediators in wound healing. Surg. Clin. North Am. 2003; 83(3): 483 - 507.
41. Herouy Y, Trefzer D, Hellstern MO, Stark GB, Vanscheidt W, Schopf E, Norgauer J. Plasminogen activation in venous leg ulcers. Br J. Dermatol. 2000; 143(5): 930 - 936.
42. Hib, J. 2001. Histología Di Fiore. En: Tejido Conectivo. Hib, J (ed) Editorial El Ateneo, Argentina, pp 35 - 45.
43. Jiménez PA, Jiménez SE. Tissue and cellular approaches to wound repair. Am J Surg. 2004; 187(5A): 56S - 64S.
44. Juhlin L. Hyaluronan in skin. J Int Med. 1997;242(1): 61 - 66.
45. Kurkinen M, Vaheri A, Roberts PJ, Stenman S. Sequential appearance of fibronectin and collagen in experimental granulation tissue. Lab Invest. 1980; 43(1): 47 - 51.
46. Lanza R, Langer R, Vacanti J. 2000. Principies of Tissue Enginering. Naughton G (ed). En: Dermal equivalents. Academic Press, New York, pp 891 - 901.
47. Laurent T y Fraser J. Hyaluronan. FASEB J. 1992; 6: 2397 - 2404.
48. Liu H, Mao J, Yao K, Yang G, Cui L, Cao Y. A study on a chitosangelatin-hyaluronic acid scaffold as artificial skin in vitro and its tissue engineering applications. J Biomater Sci Polym Ed.. 2004; 15(1): 25 - 40.
49. Lutolf M y Hubbell J. Synthetic biomaterials as instructive extracellular microenvironments for morphogenesis in Tissue Engineering. Nat Biotechnol. 2005; 23 (1): 47 -55.
50. Lynch S, Colvin R, Antoniades H. Growth factors in wound healing. Single and synergistic effects on partial thickness porcine skin wound. J. Clin. Invest. 1989; 84(2): 640 - 646.
51. Mansbridge J, Liu K, Patch R, Symons K, Pinney E. Three - dimensional fibroblast culture implant for the treatment of diabetic foot ulcers: metabolic activity and therapeuctic range. Tissue Eng. 1998; 4 (4):403 - 14.
52. MINISTERIO of SALUD of Chile. 2007. Guía Clínica Gran Quemado. Serie Guías Clínicas MINSAL N° 55.
53. Navarrete G. Histología of la piel. Rev Fac Med UNAM. 2003; 46 (4): 130 - 133.
54. Noordenbos J, Dore C, Hansbrough JF. Safety and efficacy of TransCyte for the treatment of partial - thickness burns. J Burn Care Rehabil. 1999; 20(4): 275 - 281.
55. Paul W y Sharma C. Chitosan and Alginate Wound Dressings: A Short Review. Trends Biomater. Artif. Organs. 2004; 18 (1): 18 - 23.
56. Pham H, Rich J y Veves A. Using living skin equivalents for diabetic foot ulceration. Int J Low Extrem Wounds. 2002; 1 (1): 27 - 32.
57. Sage E. Regulation of interactions between cells and extracellular matrix: a command performance on several stages. J. Clin. Invest. 2001; 107 (7): 781 - 783.
58. Schilling J. Wound healing. Surg. Clin. North Am. 1976; 56: 859.
59. Smith D, McHugh T, Phillips L, Robson M y Heggers P,. Biosynthetic compound dressing - management of hand burns. Burns1988; Incl Therm Inj. 14(5): 405 - 408.
60. Smola H, Thiekotter G y Fusenig N. Mutual induction of growth factor gene expression in by epidemial - dermal cell interaction. J. Cell Biol. 1993; 122 (2): 417 - 429.
61. Sosa A, Álvarez C, Cuenca J, Juárez E, Kuri - Harcuch W. Cir Plast. 1999; 9 (3): 126 - 129.
62. Tamariz - Domínguez E, Castro F, Kuri Harcuch W. Growth factors and extracellular matrix proteins during wound healing promoted with frozen cultured sheets of human epidemial keratinocytes. Cell Tissue Res. 2002; 307(1): 79 - 89.
63. UDL Labs. 2008. UDL Laboratories, http://www.udllabs.com (ingreso el 27 of Agosto of 2008).
64. Van Dorp A, Verhoeven M, Koerten H, Van Der Nat T - Van Der Meij TH, Van Blitterswijk C, Ponec M. Dermal regeneration in full - thickness wounds in Yucatán miniature pigs using a biodegradable copolymer. Wound Repair Reg. 1998; 6(6): 556 - 568.
65. Veves A, Falanga V, Armstrong D, Sabolinski M. Graftskin, a human skin equivalent is effective in the management of noninfected neuropathic diabetic foot ulcers: a prospective randomized multicenter clinical trial. Diabetes Care 2001 ; 24(2): 290 - 295.
66. Wedmore I, McManus J, Pusateri A, Holcomb J. A special report on the chitosan - based hemostatic dressing: Experience in current combat operations. J Trauma. 2006; 60(3): 655 - 658.
67. WHO. 2008. World Health Organization. http://www.who.int/en (ingreso el 20 of Agosto of 2008).
68. Witte M y Barbul A. Role of nitric oxide in wound repair. Am. J. Surg. 2002; 183(4): 406 - 412.
69. Young ME, Acevedo C, Weinstein C, Brown D, Tapia, S, Albornoz F. Memoria Descriptiva Solicitud of Patente para el Sistema of Implante Integrado. Junio 2006.

### Brief Description of the Drawings

Figure 1. Skin Structure. (1) Hair, (2) sweat glands, (3) basal lamina, (4) blood vessels (Hib, 2001)
Figure 2. Histology and immunohistochemistry of microencapsulated cells. A. a cell in a homogeneous area of fibrin stained with methylene blue (microencapsulation day); B. cell in metaphase located close to the fibrin which is stained with erythrosin (48 hours after microencapsulation); C. three cells embedded in the fibrin gel (24 hours after microencapsulation D. a pair of cells located in the pores of the fibrin matrix (48 hours after microencapsulation); E. group of cells located within the fibrin gel (96 hours after microencapsulation); F. expansion of the cells cluster of E (arrows) where a cell in metaphase is observed; from G. to I.: Immunohistochemistry of a cell cluster growing in a fibrin gel; G. control with Arteta trichrome staining; H. immunolocalization of cytokeratin and I. immunolocalization of vimentin (Acevedo C. et al, Bioprocess Biosyst Eng., 32(3):341-51, 2009).

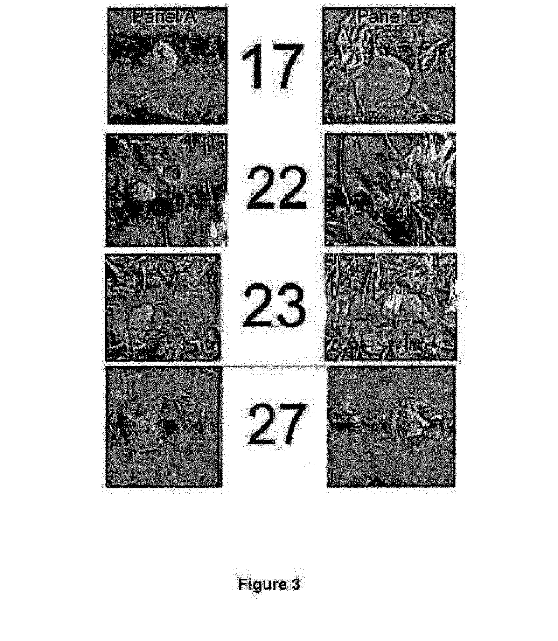

Figure 3. Study of selected clots stability (F17, F22, F23, F27). Panel A: appearance at the beginning. Panel B: appearance at three days post-incubation at 37°C.
Figure 4. Growth curves of human fibroblasts, grown on the mentioned surfaces. Statistically significant differences in growth with respect to day 0 are quoted (*) (ANOVA, p < 0.05).

## Claims

1. A process for the preparation of an autologous fibrin gel or from compatible blood for cell vehiculization and proliferation, **CHARACTERIZED in that** comprises the steps of:
a) Taking an amount of blood from the own patient, at a rate of 200 DL per cm of implant to be prepared, using as anticoagulant sodium citrate at a concentration of 0.09 g per ml of blood;
b) Separating the citrated plasma by centrifugation at 3000 rpm for ten minutes at 4 °C. Re-suspending cells to be vehiculized in the plasma obtained in the above step;
c) Adding to the suspension obtained in the previous step a solution of CaCl₂ at 10% for forming the gel, either as an isolated clot or within a porous matrix. Culturing the gel in an incubator at 37°C in humidified atmosphere and at 5% of CO₂, for cell growth purposes.

2. Process according to claim 1, **CHARACTERIZED in that** the blood sample of the patient or compatible blood is maintained no coagulated with sodium citrate.

3. Process according to claim 2, **CHARACTERIZED in that** the blood sample from the patient or compatible blood, is maintained preferably no coagulated using sodium citrate

4. Process according to claim 3, **CHARACTERIZED in that** the blood sample of the patient or compatible blood is maintained no coagulated, preferably using sodium citrate in a ratio of 2.5 ml of blood with about 0.05 to 0.1 g/mL sodium citrate.

5. Process according to claim 3, **CHARACTERIZED in that** the blood sample of the patient or compatible blood is maintained no coagulated, preferably using sodium citrate in a ratio of:
• 100µl of citrated plasma with 100µl of solution of CaCl₂ 30mM.
• 100µl of citrated plasma with 150µl of solution of CaCl₂ 30mM
• 100µl of citrated plasma with 200µl of solution of CaCl₂ 30mM
• 100µl of citrated plasma with 75µl of solution of CaCl₂ 40mM.
• 100µl of citrated plasma with 100µl of solution of CaCl₂ 40mM.
• 100µl of citrated plasma with 150µl of solution of CaCl₂ 40mM.
• 100µl of citrated plasma with 200µl of solution of CaCl₂ 40mM.
• 100µl of citrated plasma with 50µl of solution of CaCl₂ 50mM.
• 100µl of citrated plasma with 75µl of solution of CaCl₂ 50mM.
• 100µl of citrated plasma with 100µl of solution of CaCl₂ 50mM.

6. Process according to claim 1, **CHARACTERIZED in that** the amount of blood collected from the patient or compatible blood is between about 2.5 mL of 250 ml.

7. Process according to claim 6, **CHARACTERIZED in that** about 2.5 to 250 ml in sodium citrate is subjected to centrifugation at 453 x g for 5 minutes.

8. Process according to claim 7, **CHARACTERIZED in that** the product of the centrifugation should be frozen at -20°C to -86°C until use.

9. Process according to claim 1, **CHARACTERIZED in that** for the gel of autologous fibrin or from compatible blood is required to prepare a polymer matrix of chitosan-hyaluronic acid gel.

10. Gel of autologous fibrin or from compatible blood, **CHARACTERIZED in that** it is obtained by the process of claim 1.

11. Use of autologous fibrin gel or of compatible blood, **CHARACTERIZED in that** it is used for surgical, sealing of wounds or skin lesions of cartilage lesions, gingival or bone, free of biological, replacement, integration of surgical sutures and any application in the field of the engineering of tissue.
